# EUROPEAN PATENT SPECIFICATION

(11) **EP 0 642 368 B1**
(45) Date of publication and mention of the grant of the patent: **27.01.1999**
(21) Application number: 93909278.9
(22) Date of filing: 06.04.1993
(51) Int. Cl.: A61N 1/39

(54) **APPARATUS FOR TREATMENT OF VENTRICULAR TACHYCARDIA USING A FAR-FIELD PULSE SERIES**
GERÄT ZUR BEHANDLUNG VON HERZKAMMER-TACHYKARDIEN MITTELS REIHE VON ENTFERNTENFELD-IMPULSEN
APPAREIL DE TRAITEMENT DE LA TACHYCARDIE VENTRICULAIRE AU MOYEN D'UNE SERIE D'IMPULSIONS DE CHAMP LOINTAIN

(30) Priority: 06.04.1992 US 863738
(43) Date of publication of application: 15.03.1995
(73) Proprietor: ANGEION CORPORATION, Brooklyn Park, MN 55428-1088 (US)
(72) Inventor: KROLL, Mark, W., Simi Valley, CA 93065 (US); ADAMS, Theodore, P., Edina, MN 55435 (US)
(74) Representative: Schütz, Peter, Dipl.-Ing.
(86) International application number: PCT/US93/03267
(87) International publication number: WO 93/19809

(56) References cited:
- EP-A- 0 095 726
- FR-A- 2 528 708
- US-A- 3 942 536
- US-A- 4 403 614
- MEDICAL & BIOLOGICAL ENGINEERING vol. 6, no. 3, March 1968, (GB) pages 167 - 169 J. E. W. KUGELBERG 'ventricular defibrillation with double square pulse'

## Description

### BACKGROUND OF THE INVENTION

### 1. Field of the Invention

The present invention pertains to an apparatus used to treat human heart arrythmia conditions. More particularly, the present invention relates to an implantable apparatus for treating ventricular tachycardia.

### 2. Description of the Background Art

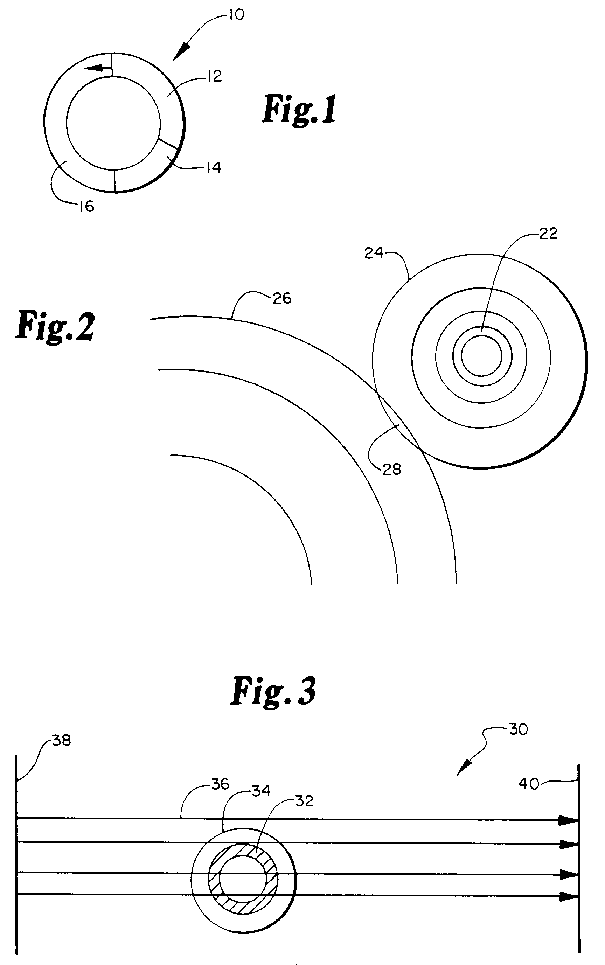

Ventricular tachycardia is a racing of the electrical signals within one or more regions of the ventricular chambers of the myocardium, i.e., the heart. Currently, the most accepted explanation for ventricular tachycardia is that a race condition is somehow started in a closed loop of myocardium with a diameter on the order of 1 cm, as illustrated in Figure 1. In this closed loop, the path of electrical cell stimulation is essentially circular, with a path length (circumference) sufficient to permit a given cell to repolarize, or to recover, from the previous stimulation, before the wave of cell stimulation comes around the circle the next time. In this way, the electrical cell stimulation within the closed loop is self sustaining and races ahead of the normal electrical cell stimulation of the myocardium.

In biomedical terminology, such a closed loop is described as a re-entrant loop, terminology which generically identifies a situation where a path of adjacent-cell stimulation closes on itself, or reenters a previous path. In the loop case, the activated cells continue to activate cells in a circular rotation in continuous fashion. Immediately behind the cells just stimulated are cells that are fairly refractory or resistant to stimulation having not fully recovered from activation. Farther behind them are cells that are fully recovered (repolarized), and hence, are amenable to stimulation or reactivation.

This kind of single-source ventricular tachycardia as shown in Figure 1 generates a rather consistently shaped electrical signal, and hence, is referred to as a monomorphic ventricular tachycardia. If there is more than one source or more than one re-entrant loop involved in the tachycardia, then the arrhythmia is referred to as a polymorphic ventricular tachycardia.

Presently, the most common and accepted method for treating ventricular tachycardia with an implantable cardioverter defibrillator (ICD) is to deliver a series of pacing pulses at a location of the heart remote to the re-entrant loop. This series of tachycardia pacing pulses is selected to have a rate that is slightly higher than the rate of the ventricular tachycardia, and each pulse has an energy of less than about 50 microjoules. The tachycardia pacing pulses are delivered through conventional pacing electrodes that are essentially small point sources. The choice of using small point sources to deliver the tachycardia pacing pulses is made because the energy required to stimulate the closest neighboring cells is smaller. The activation pulse wave then spreads through the myocardium towards the re-entrant loop. In theory, one of the tachycardia pacing pulses should arrive at the re-entrant loop in the correct phase and thereby stimulate the cells of the re-entrant loop that have recovered fully from activation. If this happens, then these cells will be refractory to continued loop activation, and hence, the ventricular tachycardia will terminate.

The basic concept of anti-tachycardia pacing was invented by Zacouto in the late 1960s. There have been many patents on traditional anti-tachycardia pacing. Examples are: U.S. Patent No. 4,312,356 to Sowton; U.S. Patent No. 4,390,021 to Spurrell; U.S. Patent No. 4,398,536 to Nappholz; U.S. Patent No. 4,408,606 to Spurrell; U.S. Patent No. 4,488,554 to Nappholz; and U.S. Patent No. 4,577,633 to Berkovitz.

Some of the early anti-tachycardia pacing devices were similar to conventional pacemakers and had no ability to deliver conventional defibrillation countershock in addition to the anti-tachycardia pacing pulses. Due to an occasional iatrogenic deterioration of the ventricular tachycardia, such as an acceleration of the tachycardia that requires a defibrillation countershock, anti-tachycardia pacing therapy is currently only delivered by ICD systems which incorporate the anti-tachycardia pacing as a programmable option for the ICD.

The most typical treatment modalities for anti-tachycardia pacing have used a burst of ten pacing pulses delivered at a rate slightly higher than the ventricular-tachycardia rate. The choice of a higher rate of pacing is made because the anti-tachycardia pacing pulses will vary in phase with respect to the ventricular tachycardia, and thus, there is an increased opportunity for the pacing wave to arrive at a depolarized portion of the re-entrant loop before the pacing wave is itself annihilated. In an effort to improve the effectiveness of anti-tachycardia pacing, pulse-rate scanning is sometimes employed in an effort to hit the correct rate. In scanning, the pacing value is increased with every burst. In another technique designed to improve the effectiveness of anti-tachycardia pacing, the pacing rate is sometimes increased or ramped during the anti-tachycardia pacing bursts as disclosed in US 4398536.

There are several timing problems that prevent a 100% success rate with existing anti-tachycardia pacing techniques. The major problem is that the treatment pulse wave must arrive at the nearest side of the re-entrant loop while it is in repolarization, or amenable to activation. Before it can do this, the treatment wave must progress through myocardium without being blocked by the activation waves emanating from the loop itself. Such blocking can happen because the re-entrant loop is generating an expanding activation wave throughout the myocardium in a fashion similar to the waves from a pebble dropped into the center of a smooth pond. This problem is illustrated in Figure 2 which shows the treatment wave at the lower left emanating from a pacing electrode in this example. In an intermediate region, the treatment wave competes with the wave launched by the re-entrant loop responsible for the tachycardia, and has a fair probability of being blocked.

Primarily because of these critical timing constraints, anti-tachycardia pacing techniques have been reported to work only 50% to 90% of the time in cases of monomorphic ventricular tachycardia. Unfortunately, the series of anti-tachycardia pacing pulses sometimes not only fail to completely treat the arrythmia, but also may cause a higher-rate monomorphic ventricular tachycardia, or pulse-rate acceleration. This can result in patient unconsciousness, which requires a painful higher-energy defibrillation shock in order to terminate the arrythmia. Added to these problems for the monomorphic case, is the fact that for cases of polymorphic ventricular tachycardia with multiple loops (or sources), the prior art anti-tachycardia pacing therapy has essentially no chance of working.

The existing situation with respect to anti-tachycardia pacing treatment can be summarized as follows. The high pulse rate in monomorphic tachycardia is a consequence of an electrical cell stimulation signal circulating in a small-diameter closed loop at some location on the myocardium. The anti-tachycardia pacing therapy delivers a short series of pacing pulses at a rate higher than that of the tachycardia. The intent of the higher rate pacing therapy is to have the arrival of each wave coincide with the repolarized (or stimulation-amenable) zone present at the nearest edge to have a fair probability of success by arriving at just the right instant. The point of using a burst of pulses, rather than a long series, is to provide a quiet interval after therapy during which dominance can be reestablished by waves coming from the proper pacemaker (whether from the heart's natural pacing center, or from the electrodes of a prosthetic pacemaker). The problem is that anti-tachycardia pacing often yields indifferent results, even in the simplest monomorphic case. The pacing pulse waves may not arrive at the right time, may be annihilated by tachycardia waves, and may induce even more serious arrythmia conditions.

In addition to the standard anti-tachycardia pacing treatment that uses pacing electrodes on a standard cardiac catheter to deliver tachycardia pacing pulses, there is another accepted technique for treating ventricular tachycardias. This treatment, known as cardioversion, involves the delivery of a single electrical pulse to the entire myocardium through relatively large-area defibrillation electrodes. Such a cardioversion pulse usually has an energy in the neighborhood of 1-5 joules. This value is well above that of a anti-tachycardia pacing pulse, which is typically about 50 microjoules, but below that of a typical defibrillation pulse, which is typically greater than 5 joules. In essence, the cardioversion treatment is somewhat analogous to lighting a back-fire in the entire heart to stop a forest fire.

The existing technique for cardioversion is to use an ICD to deliver a single electrical pulse to the myocardium that it essentially the same as existing defibrillation pulses, only of a smaller initial voltage. In other words, the cardioversion pulse has the same shape and duration as existing defibrillation pulses, only an initial voltage of about 150 volts is used, rather than a typical initial voltage of between 600-750 volts for a defibrillation countershock. Because the objective of existing cardioversion treatment is to immerse all, or substantially all of the myocardium, in an electric field similar to, but less intense than, a defibrillation pulse, the single cardioversion pulse is delivered through defibrillation electrodes that have a larger surface area than pacemaker electrodes. Once the single cardioversion pulse is delivered, the ICD waits a couple of seconds for the heart to "recover" and then returns to a sensing mode to determine if the arrhythmia condition still exists. If the cardioversion pulse was unsuccessful in returning the heart to normal cardiac rates, a standard treatment is to immediately deliver a defibrillation countershock pulse to recover normal heart activity.

While the idea of starting such a small backfire to extinguish ventricular tachycardia has success in some situations, existing clinical data suggests that cardioversion is successful in only about 50-70% of ventricular tachyarrhythmia conditions. In addition to being a very painful therapy, cardioversion can produce a deterioration of the tachycardia as often as 25% of the time the therapy is used. Finally, cardioversion is a relatively energy inefficient therapy compared to anti-tachycardia pacing in terms of the energy storage requirements necessary to deliver this type of pulse, particularly if several tachycardia incidents are treated in a short period of time.

Presently, the accepted practice is to deliver only one stimulation pulse at a time through the defibrillation electrodes, regardless of whether that stimulation pulse is a cardioversion pulse or a defibrillation countershock. The inventors are aware of only two prior attempts to deliver more than one electrical pulse at a time through the defibrillation electrodes.

In an abstract presented at the American Heart Association meeting in 1991, Shenasa reported applying a series of very low voltages, under one volt, to defibrillation electrodes. *Circ.* Vol. 84, No. 4, p. II-426 (Oct. 1991). He characterized these voltage as "sub-threshold stimulation" levels, and applied them at extremely high rates (50-millisecond pulses at the rate of 1200/minute). Shenasa's philosophy was to numb the myocardial cells near the electrodes in order to make these cells less likely to sustain the re-entrant loop of the ventricular tachycardia. He reported only a 52% success rate in treating tachycardia arrhythmias using this methodology.

U.S. Patent No. 4,996,984 to Sweeney discloses the use of a pair (or more) of pulses for defibrillation. The disclosure of this patent addresses only fibrillation arrythmia conditions, and not tachycardia arrhythmias. The stimulation pulses described have just slightly less energy than conventional defibrillation pulses, on the order of 10 joules, and defibrillation voltage levels, on the order of about 600-750 volts. The use of a train of defibrillation pulses by Sweeny was apparently inspired by the 20-year-old work of the Swedish researcher Kugelberg, who also used trains of high-energy pulses to achieve defibrillation (see also the article "Ventricular Defibrillation With Double-Square Pulse" by Kugelberg in Med. & biol. Engineering, vol. 6, pages 167-169, Pergamon Press, 1968).

From EP-A-095 726 a method and apparatus for controlling cardiac ventricular tachyarrhythmias is known in which two pairs of electrodes are implanted into the heart such that their electric fields cross each other when the electrodes are being excited in a time sequence whereby the first pulse is applied to the first pair of electrodes and thereafter the second pulse is applied to the other pair. In this way a more uniformed distribution of currents within the ventricular myocardium is achieved. The pulses have a truncated-exponential waveform of about 1 through 5 milliseconds and having an amplitude between 100 and 1000 volts, the intervals between the pulses being between 0.1 and 2 milliseconds.

In FR-A-2 528 708 a device for delivering a series of defibrillation pulses is described which pulses are effective to modify a detected arrhythmia from a heavily deorganized condition into a better organized condition which can be more easily treated.

While existing anti-tachycardia therapies offer some hope for successful treatment of tachycardia arrhythmias without resorting to defibrillation, the problem with the existing therapies is that they are effective in only about 50% of the cases, and in as many as 25% of the cases, the anti-tachycardia therapy may actually cause deterioration of the arrhythmia into a condition that requires defibrillation.

The object of this invention is to improve an implantable apparatus for ventricular tachycardia treatment with a higher success rate and a lower complication rate.

This object is to be solved by the features of claim 1. Particular embodiments of the invention are manifested by the features of the subclaims.

### SUMMARY OF THE INVENTION

The present invention is an implantable apparatus for treating ventricular tachycardia by delivering a series of anti-tachycardia stimulation pulses, each of which has energy values of less than 5 joules, and at least one of which is a far-field pulse. As defined by the present invention, a far-field pulse is a short, relatively high-voltage pulse delivered through at least two far-field electrodes. The series of anti-tachycardia stimulation pulses are preferably delivered by an implantable cardioverter defibrillator (ICD) having at least two relatively larger-surface-area defibrillation-style far-field electrodes having a relatively large inter-electrode distance, and a pair of smaller-surface-area pacing-style electrodes having a relatively smaller inter-electrode distance. Each series of anti-tachycardia stimulation pulses is delivered as a committed burst of pulses without any intervening monitoring, and with one or more bursts being delivered by the ICD in response to the sensing of a ventricular tachycardia. By using the large surface area defibrillation-style electrodes to deliver at least one of the anti-tachycardia pulses, the present invention induces a far-field stimulation of the myocardium in an effort to control and extinguish the ventricular tachycardia.

A typical far-field pulse of the present invention has an amplitude of between about 5 to 200 volts, an electrical energy between about 0.01 and 5 joules and a pulse duration of preferably less than about 3 milliseconds. The voltage is chosen to be high enough to stimulate a large portion, but not necessarily all, of the myocardium directly, without consuming high levels of energy. The pulse duration is chosen to approximate the chronaxie time for far-field stimulation of depolarized heart cells. This approximate match minimizes the delivered energy required per pulse. The timing of the rate of delivery of the far-field pulses of the present invention is similar to the timing used in existing anti-tachycardia pacing. The entire burst of the series of stimulation pulses is delivered in less than about 5 seconds and is a committed sequence of stimulation pulses in the sense that no intervening monitoring of cardiac function is performed between stimulation pulses.

According to the invention, an implantable apparatus for treating ventricular tachycardia in a human patient, comprises: at least a pair of far-field electrodes and at least a pair of pacing electrodes. Said far-field electrodes having a combined surface area that exceeds about 1 square centimeter and an inter-electrode distance of greater than about 2 centimeters; means for sensing a ventricular tachycardia in the human patient; and means for delivering a series of two or more anti-tachycardia electrical stimulation pulses in response to the ventricular tachycardia, each stimulation pulse having an energy value of less than about 5 joules to the two or more electrodes in a committed burst of less than about 5 seconds, at least one of the stimulation pulses being a far-field pulse delivered through the far-field electrodes and having an energy value between about 0.01 joule and 5 joules and an amplitude between about 5 volts and 200 volts.

It is important for an appreciation of this invention, to understand the difference between existing anti-tachycardia pacing and cardioversion techniques, and the far-field stimulation technique of the present invention.

In anti-tachycardia pacing stimulation, a low-voltage pulse of less than about 5-10 V is delivered into a pacing-style electrode, typically having an electrode surface area of much less than 1 cm² and an inter-electrode distance of less than about 2 centimeters. This pulse "breaks down", or renders conductive, the membranes of a handful of neighboring cells adjacent the pacing style electrodes so that they are activated (depolarized). These cells then stimulate more cells with which they are in direct contact. This cell-to-cell stimulation is not perfectly understood, but is thought to involve both ionic currents and capacitive coupling (displacement currents). The activation wave that is created by this point source cell-to-cell stimulation then moves through the heart at a velocity of about 100 cm/sec. An analogy is the lighting of a horizontal sheet of paper by igniting one corner. The corner is easy to ignite and the flame then spreads as an activation wave through the paper.

In cardioversion stimulation, a single moderately-high voltage pulse of about 150 V is delivered into a defibrillation-style electrode, typically having a surface area of greater than about 1 cm² and an inter-electrode distance of greater than about 2 centimeters. The single cardioversion pulse has the same shape and duration as existing defibrillation pulses. Unlike the anti-tachycardia pacing pulses, the objective of existing cardioversion treatment is to immerse all, or substantially all of the myocardium, in an electric field similar to, but smaller than, a defibrillation pulse.

In the present invention, a far-field pulse series is used to treat the ventricular tachycardia. Unlike existing anti-tachycardia pacing treatments, the present invention delivers at least one far-field pulse in the series that is a relatively larger pulse routed through the defibrillation-type far-field electrodes. As will be described, the far-field electric field created by delivering the far-field pulse through at least two far-field electrodes having a relatively larger surface area and a relatively longer inter-electrode distance generates an electric field that simultaneously covers a larger portion of the myocardium and then quickly repeats this process at a rate faster than the rate of the ventricular tachycardia contractions. Unlike existing cardioversion treatments, the present invention delivers a committed series of pulses without any intervening monitoring, and not just a single pulse.

In essence, the theory behind the far-field pulse series is substantially different than the theories for either of the existing tachycardia treatments. Instead of trying to extinguish the ventricular tachycardia using a backfire-like wave of small stimulation pulses delivered from a pair of relatively close point source electrodes, or completely dousing the myocardium with a single smaller fibrillation-like pulse delivered from a pair of relatively distant large-area electrodes and then waiting for the normal stimulation of the heart to be reestablished. the present invention uses a series of stimulation pulses, at least one of which is a far-field pulse, to essentially simultaneously extinguish any re-entrant loops causing the ventricular tachycardia and also assist the heart in reestablishing normal sinus rhythm.

Thus, in the far-field stimulation of the present invention, the myocardial cells are simultaneously activated. This has a major advantage for dealing with monomorphic ventricular tachycardia, in that it removes (or at least minimizes) the timing problems of conventional anti-tachycardia pacing. This is because the far-field stimulation spreads not by activation of cells, but by electromagnetic wave propagation moving at a large fraction of the speed of light. This constitutes simultaneous stimulation as far as biological systems are concerned. More importantly, the far-field stimulation is not dependent on finding a path through inactivated (depolarized) cells. The desired stimulation spreads through electrical conduction, and not through cell-to-cell activation, which requires a path of cells that were not previously activated.

Hence, the far-field stimulation of the present invention does not suffer from the two timing problems that seriously limit the effectiveness of prior art anti-tachycardia pacing. First, it is not annihilated by activation waves emanating from the loop; and second, it need not first make contact with the re-entrant loop at a depolarized section. Also, unlike the single pulse techniques of the prior art cardioversion therapies, the present invention uses a committed series of anti-tachycardia stimulation pulses without any intervening monitoring or waiting periods in order to optimize the chances of destructively interfering with the re-entrant loop of the ventricular tachycardia so as to extinguish the arrhythmia.

It is important to note that the far-field stimulation need not be so strong as to stimulate the whole heart. If the strength of the pulses is sufficient to stimulate directly a relatively large portion of the functional myocardial tissue, then there is a significantly increased chance of terminating the ventricular tachycardia. Preferably, the stimulated region will be at least 50% of the functional myocardial tissue. If the generating loop is within the stimulated region, then the tachycardia may be terminated due to the electrical conduction of the far-field pulses within that region. If the loop is outside of this region, then the far-field stimulation still has a much improved chance of termination since there are more and broader pathways for delivering the stimulation to the loop via cell-to-cell stimulation from the region which was stimulated by the far-field pulses.

There are many ways to deliver the far-field stimulation pulses to the larger surface area electrodes in accordance with the present invention. One technique is to use the main (defibrillation) capacitor in the implantable cardioverter defibrillator (ICD), charging it up to a relatively high voltage (5 to 200 volts) and delivering a short (about 1 millisecond) pulse from that capacitor. The advantage of this approach is that no additional capacitors are needed.

Another technique would be to use a separate smaller capacitor to deliver the far-field stimulation pulses. The advantage of this approach is that the additional capacitor may be charged to a lower voltage than that of the main capacitor. This would allow the main capacitor to be charged to a full voltage as a backup measure in case the far-field stimulation deteriorated the tachycardia condition. This additional capacitor value may also be chosen to deliver pulses of more energy efficiency to the heart. For example, the additional capacitor could have a value of some 15 to 20 microfarads, which is a small value compared to those of typical defibrillation capacitors of 140 to 180 microfarads.

Several alternate embodiments of the anti-tachycardia pulse trains used by the present invention exist. In each of them, the one-capacitor or the novel two-capacitor apparatus may be used to deliver the desired pulses.

In the first of the alternate embodiments, a relatively high-voltage anti-tachycardia far-field stimulation pulse is delivered to the far-field electrodes, with the interval between it and the last preceding contraction pulse from the tachycardial malfunction being shorter than the intervals between the train of tachycardia pulses. The far-field pulse is then followed by a train of ten or fewer pacing pulses delivered to pacing electrodes of a cardiac catheter, with the interval between the first of these and the far-field pulse, as well as their mutual intervals being at least as short as the first specified interval.

The next alternate embodiment of the present invention employs two relatively high-voltage far-field pulses in place of one. The interval between the two far-field pulses is the same as the first-specified interval in the previous embodiment, and the subsequent intervals of the pacing pulses are specified the same as those in the previous embodiment.

The last alternate embodiment is like the one just described, except that the interval between the two high-voltage far-field pulses is different from the intervals of the pacing pulses that follow.

### BRIEF DESCRIPTION OF THE DRAWINGS

Figure 1 illustrates the re-entrant loop of cells that enters into a widely accepted theory of ventricular tachycardia;
Figure 2 illustrates the interaction and annihilation of a wave from a conventional antitachycardia pacing pulse by a wave from the re-entrant loop;
Figure 3 illustrates the far-field stimulation of a preferred embodiment of the present invention for tachycardia termination;
Figure 4 illustrates schematically a circuit used for generating both defibrillation pulses and the antitachycardia stimulation pulses of a preferred embodiment of the present invention;
Figure 5 illustrates schematically a circuit of a preferred embodiment of the present invention used for generating defibrillation pulses with one capacitor and the anti-tachycardia stimulation pulses with an additional capacitor optimized for the purpose;
Figure 6 illustrates the placement and configuration of alternative embodiments of the electrodes of a preferred embodiment of the present invention; and
Figure 7 illustrates schematically a circuit used to produce the stimulation pulse series in an embodiment of the present invention that utilizes both far-field electrodes and pacing electrodes.

### DETAILED DESCRIPTION OF THE PREFERRED EMBODIMENT

The following detailed description, when considered in connection with the accompanying drawings in which like reference numerals designate like parts throughout the figures, describes some of the embodiments of the present invention.

Figure 1 illustrates a re-entrant loop 10 of cells of the myocardium that enters into a widely accepted theory of ventricular tachycardia, the loop including in sequence activated cells 12, refractory cells 14 that have not yet recovered from activation, and depolarized cells 16 that can again be activated. It will be appreciated that a generally accepted definition of ventricular tachycardia is ventricular tachycardia contractions at a sustained rate of between 150 and 240 bpm.

Figure 2 illustrates schematically the geometrical pattern 20 of the interaction of a re-entrant loop 22 having a wave such as 24 interacting with a wave such as 26 that is emanating from the pacing electrodes of a conventional catheter in the conventional antitachycardia pacing therapy, with wave annihilation occurring in the region 28. For the reasons discussed above, the chances that the anti-tachy pacing wave 26 will succeed in blocking the re-entrant loop wave 24 is only about 50-80%.

Figure 3 illustrates schematically the geometrical pattern 30 of the stimulation of a re-entrant loop 32 with a wave 34 emanating from it, by an electric field represented by lines of force such as 36, said electric field produced by far-field electrodes 38 and 40 in accordance with the present invention, and being relatively unaffected by the wave 34.

In far-field stimulation, an electric field of about 5 V/cm is generated within a region of the heart by each of the far-field pulses of the present invention. The cylindrical heart cells (myocytes) are about 25 µm in diameter and 75 µm in length. The cells have a membrane with a thickness of about 8 nm. The electric field of 5 V/cm is generated parallel to the axis of the cell. The voltage drop along the length of the cell is:${\text{V}}_{\text{d}} \text{= (5 V/cm) (75 µm) = 37.5 mV}$

Intuitively, this does not seem like enough voltage to break down a cell membrane that swings by +/- 100 mV during depolarization. However, the insulating nature of the membrane, coupled with its conductive interior serves to concentrate this voltage drop through the membrane. Before membrane breakdown and cell activation, the internal cell potentials will be homogeneous. In other words, the membranes at each end of the cell must withstand the full voltage drop applied to the whole cell. Thus, each membrane end will see:$\text{(37.5 mV / 2) = 18.75 mV}$ for a field of:$\text{E = (18.75 mV / 8 nm) = 2,340 kV/m}$ which is a sufficient field to break down the membrane and begin the cell-activation process.

The present invention also takes advantage of a subtle effect that can double this field. Instead of maintaining an isopotential cytoplasm (cell interior), the cell will try to minimize the internal field by redistributing charge. Another way of looking at this is that positive ions (mostly sodium) are attracted by the field toward the negatively charged electrode (cathode). This then increases the field across the membrane at the end where the positive sodium ions accumulate. A similar effect occurs with the negative chlorine ions at the opposite end.

It has further been found that the far-field pulses are more effectively used by the myocardium when the pulse durations are shorter than the typical pulse durations used for existing defibrillation countershocks. Presently, the pulse durations of both cardioversion and defibrillation countershock pulse is at least about 7 ms. For optimal energy utilization, however, the far-field pulse should have a duration of less than about 5 ms and, depending upon whether the ICD is optimized for stored energy or for delivered energy, the ICD should have an optimum pulse duration of between about 0.8 ms and 1.2 ms (optimizing delivered energy) or between about 4.5 and 4.8 ms (optimizing stored energy).

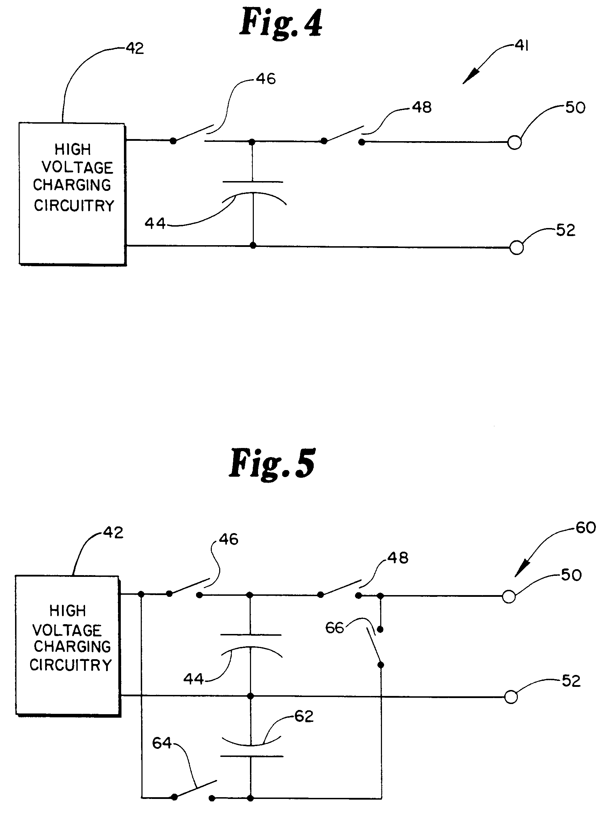

Figure 4 illustrates schematically a circuit 41 used for generating both defibrillation pulses and the antitachycardia pulses of the present invention, including a high-voltage circuitry 42 that charges capacitor 44 when switch 46 is closed; when switch 46 is subsequently opened, the capacitor 44 is isolated and can then be discharged by closing switch 48, thus delivering an energetic defibrillation pulse to cardiac electrodes 50 and 52. In a similar way, the capacitor 44 can be charged to a lower voltage and then discharged to deliver one or more lower-energy cardioversion pulses to the heart, or two or more antitachycardia pacing pulses of the present invention to the heart.

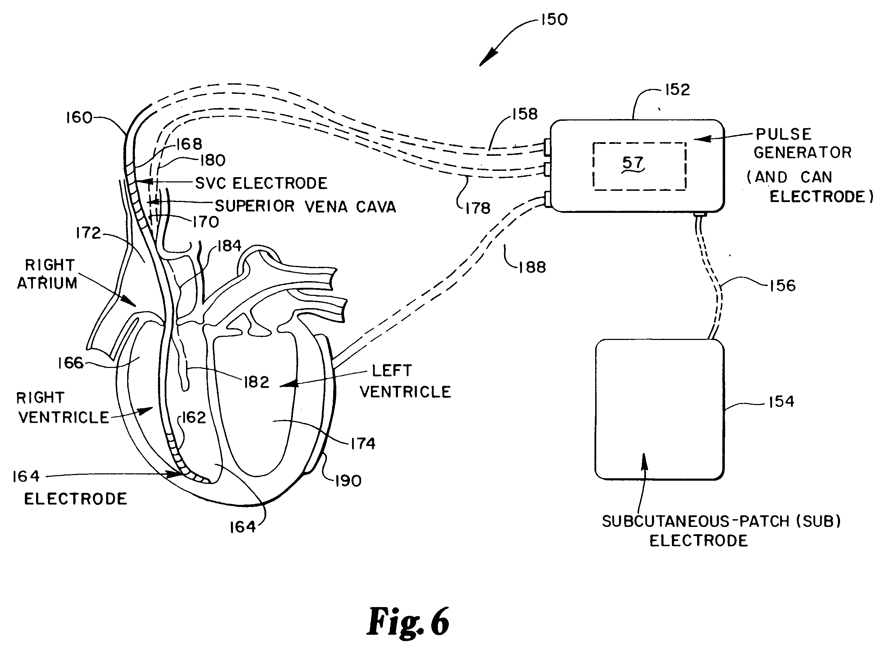

Figure 5 illustrates schematically a circuit 60 of the present invention used for generating both defibrillation pulses and the anti-tachycardia stimulation pulses of the present invention, including high-voltage circuitry 42 that charges defibrillation capacitor 44 when switch 46 is closed; when switch 46 is subsequently opened, the capacitor 44 is isolated and can then be discharged by closing switch 48, thus delivering an energetic defibrillation pulse to cardiac electrodes 50 and 52. In a similar way, the smaller anti-tachycardia capacitor 62 can be charged to a lower voltage by closing switch 64 and then discharged after opening switch 64 by closing switch 66 to deliver an optimized lower-energy cardioversion far-field pulse to the heart, a process that can be repeated to deliver two or more such far-field pulses, or to deliver two or more optimized anti-tachycardia pacing pulses of the present invention to the heart as part of a committed series of pulses.

It will be appreciated that in Figure 4, the switch 46 is closed to charge the capacitor 44 for delivery of a conventional defibrillation pulse. In Figure 5, on the other hand, switch 46 is opened to isolate the defibrillation capacitor 44, and then the switch 48 is closed to deliver the defibrillation pulse to the heart. The smaller and additional anti-tachycardia capacitor 62, on the other hand, is charged by closing switch 64. After that, switch 64 is opened to isolate capacitor 62, and then switch 66 is dosed to deliver an antitachycardia pulse to the same electrodes as before.

Figure 6 illustrates schematically and for purposes of illustration a defibrillation system 150 including a subcutaneously implanted ICD or pulse generator 157 optionally serving as a CAN electrode 152. In the embodiment shown in Figure 6, the pulse generator 157 is attached to a subcutaneously implanted SUB electrode 154 by interconnection lead 156, by interconnection lead 158 to a cardiac defibrillation-style catheter 160, by interconnection lead 178 to a cardiac pacing-style catheter 180, and by interconnection lead 188 to an epicardial patch electrode 190. The defibrillation-style catheter 160 includes a first endocardial-coil electrode 162 positioned at the apex 164 of the right ventricle 166, the coil being the RVA electrode 162, and a second endocardial-coil electrode 168 positioned in the superior vena cava 170, which is above the right atrium 172, the second coil being the SVC electrode 168. The pacing-style catheter 180 includes a first proximal 184 and a second distal electrodes 182. It will be seen from this figure that the electrodes 162 and 168 of the far-field defibrillation-style catheter are relatively larger in surface area, having a total surface area of greater than about 1 cm² and are positioned such that there is an effective inter-electrode distance of greater than about 2 cm. By effective inter-electrode distance, the present invention contemplates the distance between two theoretical point source electrodes which would generate an electric field identical to the actual physical electrode used for the far-field electrodes. In contrast to the far-field electrodes 162 and 168 of the defibrillation-style catheter, the electrodes 184 and 182 of the pacing-style catheter have relatively small surface areas, generally less than about 1 cm² and are positioned such that there is an effective inter-electrode distance of less than about 2 cm. In addition to the use of the electrodes 162 and 168 on the defibrillation-style catheter 160 as the far-field electrodes, the ICD can also use a configuration that causes the stimulation pulse current coming from the CAN electrode 152 and coming from the SUB electrode 154 to substantially to intersect the left ventricle 174, as well as the right ventricle 166. Another alternative is to use one or more epicardial patch electrodes 190 as one or more of the far-field electrodes.

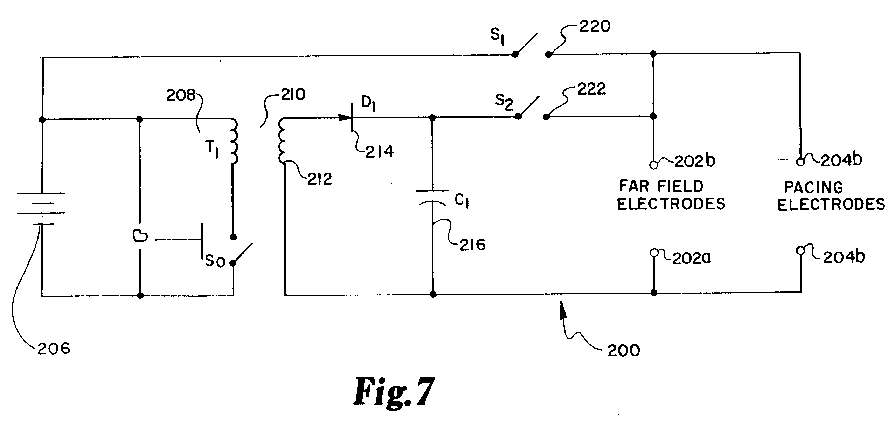

Figure 7 shows schematically a circuit 200 used to produce the stimulation pulse series in an embodiment of the present invention that utilizes both far-field electrodes 202a, 202b and pacing electrodes 204a, 204b. A battery 206 is used to supply current to the primary coil 208 of transformer 210. The secondary coil 212 of the transformer 210 produces a fly-back voltage which is rectified by diode 214 and stored in capacitor 216. For delivery of conventional pacing pulses, switch 220 is enabled which delivers a battery voltage (nominal 6 volt) to the pacing electrodes 204a, 204b. If switch 222 is enabled, it delivers the high voltage present on capacitor 216 to the far-field electrodes 202a, 202b. It will be appreciated that the capacitor of the present invention can be either a single capacitor, or a combination of capacitors having an effective capacitance required to store the necessary energy for the cardioversion pulses and defibrillation countershocks. Similarly, it will also be appreciated that the voltage of the pacing pulses can be doubled using a simple voltage doubler circuitry.

## Claims

1. An implantable apparatus (150) for treating ventricular tachycardia in a human patient, comprising
- two implantable electrodes (152,154,162,168,182,184,190), including a pair of far-field electrodes (162,168) having a combined surface area that exceeds 1 cm² and an interelectrode distance of greater than 2 cm,
- means for sensing a ventricular tachycardia in the human patient as a continuous sequence of intrinsic ventricular tachycardia contractions,
- and means (157) for delivering a series of anti-tachycardia electrical stimulation pulses in response to ventricular tachycardia, said means being arranged such as to deliver said series as a committed burst of less than 5 s of stimulation pulses with the first pulse or the first and second pulses being far-field pulses having an energy value between 0.01 and 5 Joules and an amplitude between 5 and 200 volts,
**characterized by** further comprising
- at least a pair of pacing electrodes (182,184) having a combined surface area which is less than 1 cm² and an inter-electrode distance of less than 2 centimetres;
- and a power source (42) operably connected to switching means (46) for directly producing one or more pacing pulses each having an energy value of less than 0.5 Joules, preferably less than 100 µJoules, to be delivered through said pacing electrodes (182,184) as subsequent part of the series of stimulation pulses
- without any intervening monitoring of the ventricular tachycardia contractions.

2. Apparatus as claimed in claim 1, **characterized in that** the pulse delivery means comprises: capacitor means (44,62) for storing a preselected amount of electrical energy for at least the far-field pulses; a high voltage charging circuit (42) for charging the capacitor means (44,62) to the preselected amount of electrical energy; and switching means (46) for selectively controlling a time-truncated discharge to the capacitor means (44) through the far-field electrodes (162,168) in response to the ventricular tachycardia to produce the stimulation pulses.

3. Apparatus as claimed in claim 1 or 2, **characterized in that** the far-field pulses are each arranged to have a duration of less than 5 milliseconds.

4. Apparatus as claimed in any preceding claim, **characterized in that** it is arranged to produce more than one burst of the series of stimulation pulses in order to treat the ventricular tachycardia.

5. Apparatus as claimed in any preceding claim, **characterized in that** it is arranged so that the total number of stimulation pulses produced in any burst is less than ten.

6. Apparatus as claimed in any preceding claim, **characterized in that** the continuous sequence of intrinsic ventricular tachycardia contractions as sensed by the sensing means has a rate of between 150-240 bpm.

7. Apparatus as claimed in any preceding claim, **characterized in that** the far-field pulses are arranged to be delivered from a capacitor (62) which does not store electrical energy for a defibrillation countershock pulse, the capacitor having an effective capacitance of less than 15-20 microfarads.

8. Apparatus as claimed in any preceding claim, **characterized in that** the first pulse is arranged to be delivered at an interval following a ventricular tachycardia contraction that is shorter than an interval between sequential ventricular tachycardia contractions, the subsequent stimulation pulses being arranged so that they are spaced from the first pulse and from each other by intervals at least as short as that between the first pulse and the preceding ventricular tachycardia contraction.

9. Apparatus as claimed in any preceding claim, **characterized in that** the first and second far-field pulses being delivered at an interval following a ventricular tachycardia contraction that is shorter than the interval between ventricular tachycardia contractions, and being separated by an interval at least as short as that between the first pulse and the preceding ventricular tachycardia contraction; and the second far-field pulse being arranged to be followed by a short train of pacing pulses delivered through pacing-style electrodes and being spaced from the first and second far-field pulses and from each other by intervals which are at least as short as that between the first and second far-field pulses and/or are different from that between the first and second far-field pulses.

10. Apparatus as claimed in any preceding claim, **characterized in that** the first pulse in the series of stimulation pulses is arranged to be a conventional cardioversion pulse followed by the pacing pulses delivered after an interval that is shorter than the interval between ventricular tachycardia contractions.

## Patentansprüche

1. Implantierbares Gerät (150) zur Behandlung ventrikulärer Tachykardien bei einem menschlichen Patient mit
- zwei implantierbaren Elektroden (152,154,162,168,182,184, 190), die ein Paar Fernfeldelektroden (162,168) umfassen, deren kombinierte Oberfläche 1 cm² übersteigt und deren gegenseitiger Abstand größer als 2 cm ist,
- einem Sensor zum Fühlen einer ventrikulären Tachykardie in einem menschlichen Patienten als kontinuierliche Folge innerer ventrikulärer Tachykardiekontraktionen,
- einer Einrichtung (157) zur Abgabe einer Serie elektrischer Antitachykardie-Stimulationsimpulse als Reaktion auf eine ventrikuläre Tachykardie, wobei die Einrichtung so ausgebildet ist, daß sie die Serie als weniger als 5 sec dauernder, nicht abbrechbarer Burst von Stimulationsimpulsen liefert, von denen der erste Impuls oder der erste und zweite Impuls Fernfeldimpulse mit einer Energie zwischen 0,01 und 5 Joule und einer Amplitude zwischen 5 und 200 Volt sind,
**gekennzeichnet durch** weiterhin
- mindestens ein Paar von Schrittmacherelektroden (182, 184), die eine kombinierte Oberfläche von weniger als 1 cm² und einen gegenseitigen Abstand von weniger als 2 cm haben,
- eine Leistungsquelle (42), an welche eine Schalteinrichtung (46) angeschlossen ist zur unmittelbaren Erzeugung eines oder mehrerer Schrittmacherimpulse mit jeweils einem Energiewert von weniger als 0,5 Joule, vorzugsweise weniger als 100 µJ, welche durch die Schrittmacherelektroden (182,184) als nachfolgender Teil der Serie von Stimulationsimpulsen abgegeben werden,
- ohne daß zwischendurch eine Überwachung der ventrikulären Tachykardiekontraktionen erfolgen würde.

2. Gerät nach Anspruch 1, **dadurch gekennzeichnet**, daß die die Impulse liefernde Einrichtung enthält:
eine Kondensatoranordnung (44,62) zur Speicherung einer vorbestimmten Menge elektrischer Energie für mindestens die Fernfeldimpulse,
eine Hochspannungsladungsschaltung (42) zur Ladung der Kondensatoranordnung (44,62) auf die vorbestimmte elektrische Energiemenge, und eine Schalteinrichtung (46) zur selektiven Steuerung einer zeitlich abgebrochenen Entladung der Kondensatoranordnung (44) durch die Fernfeldelektroden (162,168) in Abhängigkeit von der ventriukulären Tachykardie zur Erzeugung der Stimulationsimpulse.

3. Gerät nach Anspruch 1 oder 2, **dadurch gekennzeichnet**, daß die Fernfeldimpulse so ausgelegt sind, daß sie eine Dauer von weniger als 5 ms haben.

4. Gerät nach einem der vorstehenden Ansprüche, **gekennzeichnet durch** eine Auslegung zur Erzeugung von mehr als einem Burst der Serie von Stimulationsimpulsen zur Behandlung der ventrikulären Tachykardie.

5. Gerät nach einem der vorstehenden Ansprüche, **gekennzeichnet durch** eine solche Auslegung, daß die Gesamtanzahl von in jeglichem Burst erzeugten Stimulationsimpulsen weniger als 10 beträgt.

6. Gerät nach einem der vorstehenden Ansprüche, **dadurch gekennzeichnet**, daß die von dem Sensor festgestellte kontinuierliche Folge innerlicher ventrikulärer Tachykardiekontraktionen eine Rate zwischen 150 bis 240 Schlägen pro Minute hat.

7. Gerät nach einem der vorstehenden Ansprüche, **dadurch gekennzeichnet**, daß die Fernfeldimpulse von einem Kondensator (62) geliefert werden, in welchem nicht die elektrische Energie für einen Defibrillationsgegenschockimpuls gespeichert ist und welcher eine effektive Kapazität von weniger als 15 bis 20 µF hat.

8. Gerät nach einem der vorstehenden Ansprüche, **dadurch gekennzeichnet**, daß der erste Impuls zu einem auf eine ventrikuläre Tachykardiekontraktion folgenden Intervall geliefert wird, welches kürzer ist als ein Intervall zwischen aufeinanderfolgenden ventrikulären Tachykardiekontraktionen, und daß die nachfolgenden Stimulationsimpulse von dem ersten Impuls und jeweils voneinander um Intervalle beabstandet sind, die mindestens so kurz wie das Intervall sind, welches zwischen dem ersten Impuls und der vorangehenden ventrikulären Tachykardiekontraktion liegt.

9. Gerät nach einem der vorstehenden Ansprüche, **dadurch gekennzeichnet**, daß der erste und zweite Fernfeldimpuls zu einem auf eine ventrikuläre Tachykardiekontraktion folgenden Intervall geliefert werden, welches kürzer ist als das Intervall zwischen ventrikulären Tachykardiekontraktionen, und daß sie durch ein Intervall voneinander getrennt sind, das mindestens so kurz wie das Intervall zwischen dem ersten Impuls und der vorangehenden ventrikulären Tachykardiekontraktion ist, und daß dem zweiten Fernfeldimpuls ein kurzer Zug von Schrittmacher-impulsen folgt, welche durch die Schrittmacherelektroden übertragen werden und vom ersten und zweiten Fernfeldimpuls sowie voneinander durch Intervalle getrennt sind, die mindestens so kurz sind, wie das Intervall zwischen dem ersten und zweiten Fernfeldimpuls und/oder unterschiedlich von demjenigen zwischen dem ersten und zweiten Fernfeldimpuls.

10. Gerät nach einem der vorstehenden Ansprüche, **dadurch gekennzeichnet**, daß der erste Impuls in der Serie von Stimulationsimpulsen ein üblicher Kardioversionsimpuls ist, dem die Schrittmacherimpulse folgen, die nach einem Intervall geliefert werden, welches kürzer als das Intervall zwischen den ventrikulären Tachykardiekontraktionen ist.

## Revendications

1. Dispositif implantable (150) pour le traitement de la tachycardie ventriculaire chez un patient humain, comprenant
- deux électrodes implantables (152, 154, 162, 168, 182, 184, 190), comprenant une paire d'électrodes de champ lointain (162, 168) possédant une aire de surface combinée qui dépasse 1 cm² et une distance entre électrodes supérieure à 2 cm,
- des moyens de détection d'une tachycardie ventriculaire chez le patient humain sous forme d'une séquence continue de contractions de tachycardie ventriculaire intrinsèque,
- et des moyens (157) de délivrance d'une série d'impulsions d'électrostimulation antitachycardique en réponse à la tachycardie ventriculaire, lesdits moyens étant prévus pour délivrer ladite série sous forme d'une salve déterminée de moins de 5 secondes d'impulsions de stimulation, la première impulsion ou les première et deuxième impulsions étant des impulsions de champ lointain ayant une valeur d'énergie comprise entre 0,01 et 5 joules et une amplitude comprise entre 5 et 200 volts,
caractérisé en ce qu'il comprend en plus :
- au moins une paire d'électrodes d'électrostimulation (182, 184) possédant une aire de surface combinée qui est inférieure à 1 cm² et une distance entre électrodes inférieure à 2 cm ;
- et une source d'énergie (42) raccordée de manière opérable à des moyens de commutation (46) afin de produire directement une ou plusieurs impulsions d'électrostimulation possédant chacune une valeur d'énergie inférieure à 0,5 joules, de préférence inférieure à 100 microjoules, destinées à être délivrées par l'intermédiaire desdites électrodes d'électrostimulation (182, 184) à la suite de la série d'impulsions de stimulation
- sans aucun contrôle intermédiaire des contractions de tachycardie ventriculaire.

2. Dispositif selon la revendication 1, caractérisé en ce que les moyens de délivrance d'impulsions comprennent : des moyens de condensateurs (44, 62) pour stocker une quantité d'énergie électrique présélectionnée au moins pour les impulsions de champ lointain ; un circuit de charge à haute tension (42) pour charger les moyens de condensateurs (44, 62) à la quantité d'énergie électrique présélectionnée ; et des moyens de commutation (46) pour commander de manière sélective une décharge tronquée dans le temps vers les moyens de condensateur (44) par l'intermédiaire des électrodes de champ lointain (162, 168) en réponse à la tachycardie ventriculaire afin d'émettre les impulsions de stimulation.

3. Dispositif selon la revendication 1 ou 2, caractérisé en ce que chacune des impulsions de champ lointain est prévue pour avoir une durée inférieure à 5 millisecondes.

4. Dispositif selon l'une quelconque des revendications précédentes, caractérisé en ce qu'il est prévu pour produire plus d'une salve de la série d'impulsions de stimulation afin de traiter la tachycardie ventriculaire.

5. Dispositif selon l'une quelconque des revendications précédentes, caractérisé en ce qu'il est prévu de manière à ce que le nombre total d'impulsions de stimulation produites dans une salve quelconque soit inférieur à dix.

6. Dispositif selon l'une quelconque des revendications précédentes, caractérisé en ce que la séquence continue de contractions de tachycardie ventriculaire intrinsèque telle qu'elle est détectée par les moyens de détection possède une fréquence comprise entre 150 et 240 pulsations par minute.

7. Dispositif selon l'une quelconque des revendications précédentes, caractérisé en ce que les impulsions de champ lointain sont prévues pour être délivrées à partir d'un condensateur (62) qui ne stocke pas d'énergie électrique pour une impulsion de défibrillation électrique synchronisée, le condensateur ayant une capacité effective inférieure à 15-20 microfarads.

8. Dispositif selon l'une quelconque des revendications précédentes, caractérisé en ce que la première impulsion est prévue pour être délivrée à un intervalle suivant une contraction de tachycardie ventriculaire qui est inférieur à un intervalle séparant des contractions de tachycardie ventriculaire séquentielles, les impulsions de stimulation ultérieures étant prévues pour être espacées de la première impulsion et les unes des autres par des intervalles au moins aussi brefs que celui séparant la première impulsion de la contraction de tachycardie ventriculaire précédente.

9. Dispositif selon l'une quelconque des revendications précédentes, caractérisé en ce que les première et deuxième impulsions de champ lointain sont délivrées à un intervalle suivant une contraction de tachycardie ventriculaire qui est plus bref que l'intervalle séparant des contractions de tachycardie ventriculaire, et sont séparées par un intervalle au moins aussi bref que celui séparant la première impulsion et la contraction de tachycardie ventriculaire précédente ; et en ce que la deuxième impulsion de champ lointain est prévue pour être suivie par un bref train d'impulsions d'électrostimulation délivrées par l'intermédiaire des électrodes d'électrostimulation et espacées des première et deuxième impulsions de champ lointain et les unes des autres par des intervalles qui sont au moins aussi brefs que celui séparant les première et deuxième impulsions de champ lointain et/ou sont différents de celui séparant les première et deuxième impulsions de champ lointain.

10. Dispositif selon l'une quelconque des revendications précédentes, caractérisé en ce que la première impulsion de la série d'impulsions d'électrostimulation est prévue pour être une impulsion classique de cardioversion suivie par les impulsions d'électrostimulation délivrées au bout d'un intervalle qui est plus bref que l'intervalle séparant des contractions de tachycardie ventriculaire.
